# EUROPEAN PATENT APPLICATION

(11) **EP 4 483 936 A1**
(43) Date of publication of application: **01.01.2025**
(21) Application number: 24184286.3
(22) Date of filing: 25.06.2024
(51) Int. Cl.: A61M 35/00, A61J 1/20

(54) **DEVICE FOR MIXING A MAIN LIQUID MEDICAL SUBSTANCE WITH A SECONDARY SUBSTANCE AND RELATED MIXING SYSTEM**

(30) Priority: 26.06.2023 IT 202300013140
(71) Applicant: Crespi, Andrea, 20025 Legnano (MI) (IT)
(72) Inventor: Crespi, Andrea, 20025 Legnano (MI) (IT)
(74) Representative: Zermani Biondi Orsi, Umberto

(57) **Abstract**

A device for mixing a main medical substance with a secondary substance comprises an internally hollow main body (5) having an open end (51), through which a hermetically sealed vial (2) containing a main medical substance can be inserted, and a closed end (52), opposite the open end, comprising a dispensing body (10) or a closing element (11) which can be opened; the main body (5) comprises at least one housing area (8) within which said vial (2) can be inserted, and at least one capsule (6) containing a secondary substance to be mixed with the main substance. The vial (2) has a closure (3) with a pierceable septum (4). A perforating element (7) is arranged inside the main body (5) to perforate the at least one capsule (6) of a secondary substance and the septum (4) of the vial (2). The main body (5) further comprises, therein, a mixing chamber (9), located between the perforating element (7) and the closed end (52), in which the secondary substance is completely mixed with the main substance to make a mixed substance (26). The main body (5) has, in the housing area (8), thickening elements (16) adapted to make temporary stops to keep the at least one capsule (6) and the vial (2) in a position distant from the perforating element (7).

## Description

### Field of the art

The present invention relates to a device for mixing substances, typically for mixing a main medical substance with at least one secondary substance. In addition, the present invention relates to a system for mixing a main medical substance with at least one secondary substance comprising such a device.

In particular, the present device is particularly used in the context of medical assistance, in hospitals, health centres or mobile aid stations, in self-medication conditions or during emergency situations or simple medical assistance provided by specialised personnel to persons who need, for example, the application, on the skin, or preparation of medicines, or even in practices related to pre-operative skin preparation. Even more in particular, according to the technical field in which the present invention falls, the mixing device is adapted to store, ready for use, and to keep separate until first use medical substances which, by their very nature, cannot be stored pre-mixed for a long time because they are unstable or not compatible in the long term.

### State of the art

Currently, devices for the application of medical substances, typically liquid or both liquid and powdered, involve a pre-mixed vial of medicine which can be coupled with a dispensing cap capable of puncturing the protective film closing the vial.

There are also other dispensing devices which keep the substances to be mixed separate until the time of actual use when, by means of a perforating element with which the cap is provided, the main substance, typically liquid, contained in a vial is put into communication with the secondary powder or liquid substance contained in a separate chamber present in the cap. The cap can then be opened to withdraw the mixed substance thus obtained, or it can itself act as a dispenser.

This type of device has drawbacks mainly due to the lack of precise control over the dosage of the medicine.

Furthermore, such technologies can only work with powders and only allow the mixing of two different substances. The possibility of mixing several liquid or powder components with each other is not envisaged. Other systems use a housing placed in the cap of the primary container but are impractical and limited in available space. Such devices do not allow the customisation of ad hoc formulations. Furthermore, mixing the substances requires further mixing chambers placed separately with respect to the housing area of the cap.

Some known devices, in particular those provided with 'tunnel' perforation mechanisms currently on the market, are not accurate in dosing. In fact, they inevitably have 'dark zones', viz., unexploitable due to the particular shapes of the trigger mechanism. Areas are created where unused residues of the dosed substances can flow, with consequent incorrect final formulations and wasted substances.

Finally, the known devices do not ensure several functions at once (e.g., formulation activation, colouring, mixing of two different powders or medical substances) through a single gesture for activating the device.

### Object of the invention

In this context, the technical task of the present invention is to provide a device for mixing a main medical substance with at least one secondary substance, free of the above-mentioned drawbacks.

In particular, it is an aim of the present invention to provide a device for mixing a main medical substance with at least one secondary substance, more simply a device for mixing liquid and/or powdered medical substances, which allows to obtain, in complete asepsis and safety, a rapid mixing and subsequent dispensing and, at the same time, dosing with an outflow calibrated in terms of flow rate (viz., mils) according to the type of medical liquid dispensed on the buffer and its use.

It is also the aim of the present invention to provide a device for mixing medical substances which allows to keep separate, until the time of actual use, two or more substances to be mixed in a stable and controlled manner.

A further aim is to provide a device for mixing a main medical substance with at least one secondary substance which allows an ad hoc customisation of the final medicine formula and a precise control of the medicine dosage.

Furthermore, it is the aim of the present invention to propose a device for mixing a main medical substance with at least one secondary substance which allows the mixed substances to be fully used without waste. Another aim is to allow the mixing of a main substance with a secondary substance, either liquid or powder, and to activate, modify, colour, and supplement the basic formulation according to the intended final recipe. These and other aims are substantially achieved by a device for mixing a main medical substance with at least one secondary substance as described in one or more of the appended claims, and by a system for the same mixing comprising such a device, a vial containing a main medical substance and a capsule containing a secondary substance.

The dependent claims then define further embodiments of a device for mixing a medical primary substance with at least one secondary substance according to the present invention.

Further features and advantages will become clearer from the detailed description of a preferred, non-exclusive embodiment of a device for mixing a main medical substance with at least one secondary substance according to the invention.

### Summary

A first aspect of the present invention provides a device for mixing a main medical substance with a secondary substance comprising an internally hollow main body having an open end, through which a hermetically sealed vial containing a main medical substance can be inserted, and a closed end, opposite the open end, comprising a dispensing body or a closing element which can be opened. The main body comprising at least one housing area within which said vial can be inserted and at least one capsule containing a secondary substance to be mixed with the main substance. The vial has a closure provided with a pierceable septum. The perforating element is arranged inside the main body to perforate the at least one capsule of a secondary substance and the septum of the vial. The main body further comprises, therein, a mixing chamber, located between the perforating element and the closed end, in which the secondary substance is completely mixed with the main substance to make a mixed substance. The main body has, in the housing area, thickening elements adapted to make temporary stops to keep the at least one capsule in a position distant from the perforating element. Furthermore, the closed end of the main body adjoins the mixing chamber so that the dispensing body or the closing element is in direct contact with the mixed substance in the mixing chamber.

The perforating element comprises a base having at least one opening to make the mixed substance consisting of the main medical substance and the secondary substance flow out towards the dispensing body or the closing element.

The housing area has longitudinal ribs, made inside the main body, along an inner surface, to enable the correct positioning of the at least one capsule and the vial therein.

A porous filter is further comprised for adjusting the outlet flow of the mixed substance, which can be positioned at the base of the perforating element within the mixing chamber.

The dispensing body comprises a spongy applicator.

The dispensing body has a tilting support to ease the application of the mixture.

The spongy applicator is inclined with respect to the longitudinal axis of the device by an angle of between 30° and 50°; the tilting support is able to further incline the dispensing body, with respect to the starting position, by an angle of between 10° and 50°.

The closing element can be selected from the group comprising a pierceable membrane, a flap, a lid, a small screwable or press-on cap, a removable film, a pierceable septum or membrane.

The main body, near the closed end, may have a threaded connection.

In a second aspect, the present invention provides a system for mixing a main medical substance with a secondary substance comprising a vial containing a main medical substance, hermetically sealed and having a closure provided with a pierceable septum; at least one capsule containing a secondary substance to be mixed with the main substance; a device for mixing a main medical substance with a secondary substance. The device has the features set out above and comprises an internally hollow main body containing at least one capsule containing a secondary substance to be mixed with the main substance contained in the vial. The main body has a housing area in which at least one capsule and the vial are positioned, and a mixing chamber in which the secondary substance completely mixes with the main substance, resulting in a mixed substance. A perforating element is arranged inside the main body in a position between the housing area and the mixing chamber, to perforate at least one secondary substance capsule and the septum of the vial.

The vial and the at least one capsule are inserted in the housing area through the open end; the at least one capsule is retained by thickening elements in the housing area so as to remain in a distanced position with respect to the perforating element when the system is not in use, thus also keeping the vial at a distance from the perforating element. The vial and capsule are then pushed towards the perforating element, which thus pierces the at least one capsule and the septum of the vial when the system is in use.

The system comprises at least two capsules and at least one of the two contains a colouring substance to highlight the release of mixture during use.

The system further envisages that the vial contains a liquid main substance, and the capsule contains a powdered secondary substance.

### Brief description of the drawings

Such a description is provided with reference to the attached figures, which are also purely illustrative and therefore not limiting, wherein:
- figure 1a is a schematic side view of a first embodiment of a system for mixing a main liquid medical substance with at least one secondary substance, in accordance with the present invention, in an operating condition in which the system is not activated;
- figure 1b is a schematic side view of an alternative configuration to the first embodiment illustrated in figure 1a, which differs from the latter in the position of the thread located internally rather than on the outer surface;
- figure 2a is a schematic side view of a second embodiment of the system, which is the subject matter of the present invention, in an operating condition in which the system is not activated;
- figure 2b is a schematic side view of an alternative configuration to the second embodiment illustrated in figure 2a, which differs from the latter for the presence of a thread located outside the end fitting;
- figure 3 is the system illustrated in figure 2a in an operating condition in which the system is activated;
- figure 4 is a schematic side view of a third embodiment of the system, which is the subject matter of the present invention, in an operating condition in which the system is not activated;
- figure 5 is the system illustrated in figure 4 in an operating condition in which the system is activated;
- figures 6a and 6b depict a schematic side view of a fourth embodiment of the system which is the subject matter of the present invention, in an operating condition in which the system is not activated and in which the applicator is positioned according to a first inclination of use (figure 6a) and a second inclination of use (figure 6b);
- figure 7 is a schematic side view of a variant of the fourth embodiment illustrated in figure 6a, in an operating condition in which the system is not activated, and which differs from the system in figure 6a only for the presence of two capsules of powdered product instead of just one capsule;
- figure 8 is the system illustrated in figure 7 in an operating condition in which the system is activated;
- figure 9 is a schematic side view of a further variant of the fourth embodiment illustrated in figure 6a, in an operating condition in which the system is activated, and which differs from the system in figure 6a for the presence of two capsules of powdered product instead of just one and for the further presence of a porous filter placed between the perforating element and the mixing chamber;

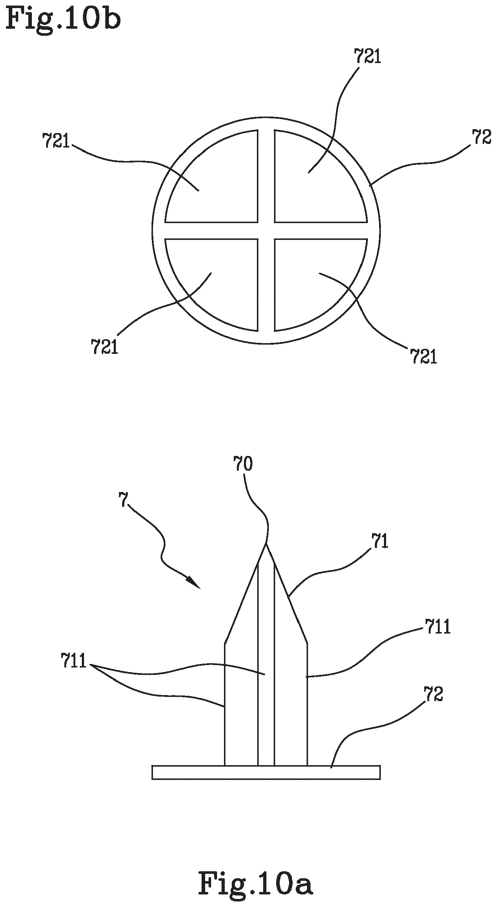

- figures 10a and 10b are a schematic side view of the perforating element present in the device which is the subject matter of the present invention;
- figure 11 is an enlargement of a detail of the main body of the device which is the subject matter of the present invention.

### Detailed description

With reference to the attached figures, a system for mixing a main medical substance with at least one secondary substance according to the present invention is collectively referred to as 1.

Preferably, the main medical substance 2' is in liquid form while the secondary substance 6' is in powdered form. However, it is also possible that the main substance is powdered and the secondary substance is liquid, or that they both have the same state, specifically both liquid.

In the preferred embodiment illustrated in the attached figures, the system 1 comprises a device 100 for mixing a main medical substance with at least one secondary substance, a vial 2 containing a main medical substance 2', preferably in liquid form, hermetically sealed and having a closure 3 provided with a pierceable septum 4, and at least one capsule 6 containing a secondary substance 6' to be mixed with the main substance. The vial 2 is preferably of the pharmaceutical type made of seamed glass, extending along a longitudinal axis X; merely by way of example, the cap can be an aluminium ring nut and central hole with septum, other alternative embodiments can be contemplated for making the closure of the vial 2.

The device 100 comprises a main body 5, preferably cylindrical and internally hollow, within which both the vial 2 and the at least one capsule 6 can be housed and which extends along the longitudinal axis X, coinciding with the longitudinal axis of the vial 2.

The secondary substance 6' may be in powdered form or, alternatively, also in liquid form. There is also a perforating element 7, adapted to perforate the at least one capsule 6 of secondary substance 6' and the septum 4 of the vial 2, to allow the main substance 2' to flow into the secondary substance 6' (or secondary substances) and mix therewith to obtain a mixed substance 26.

As mentioned, the main body 5 is internally hollow to house, therein, the vial 2 and at least one capsule 6.

In fact, the main body 5 comprises a housing area 8 therein, in which the vial 2 and at least one capsule 6 can be positioned. The main body 5 further comprises a mixing chamber 9, opposite the housing area 8.

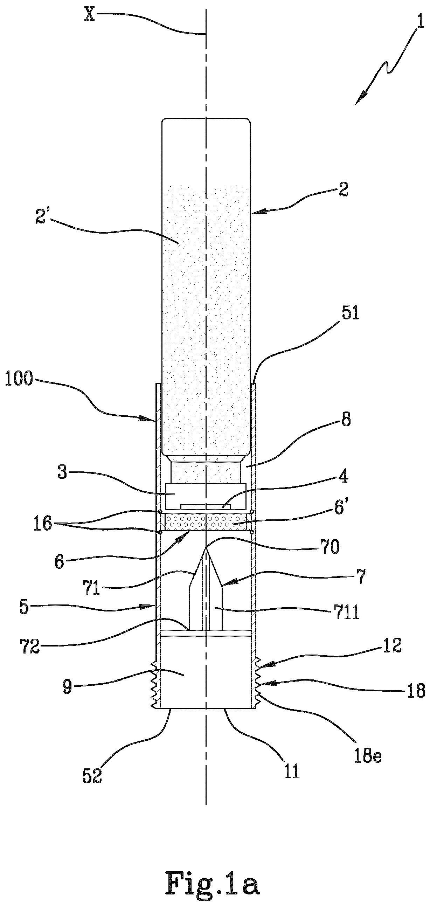

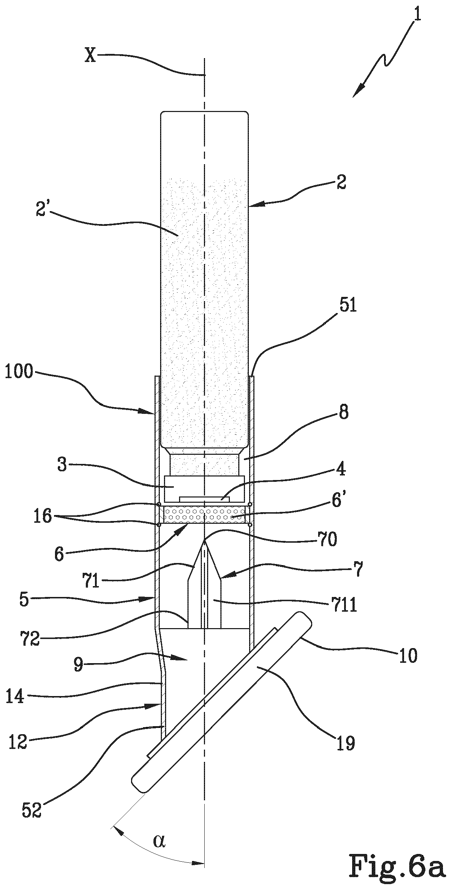

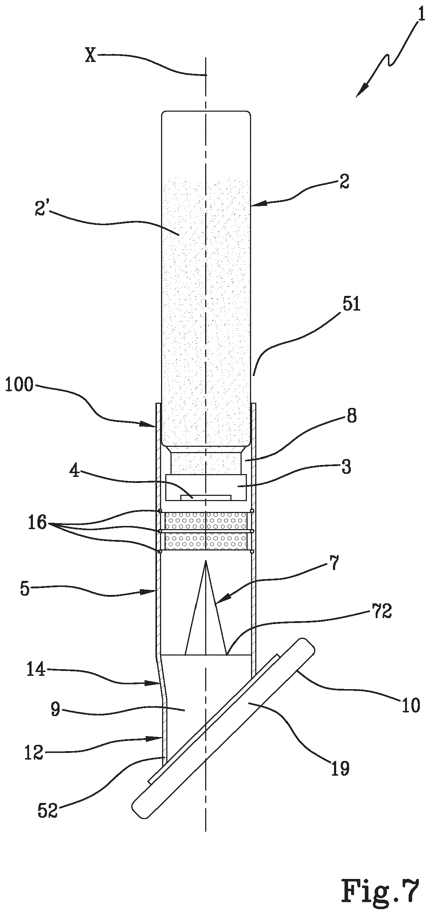

Thus, the main body 5 has an open end 51, through which the vial 2 is inserted, and a closed end 52, opposite the open end, which can comprise a dispensing body 10 (figures 6a, 6b, 7-9) or a closing element 11 which can be opened, removed or pierced (figures 1a, 1b, 2a, 2b, 3, 4 and 5). In accordance with some embodiments illustrated in figures 6a, 6b, 7-9, the closing element 11 is for example a flap, a lid, a removable film or a pierceable membrane through which the mixed substance 26 can be directly withdrawn from the mixing chamber 9. In accordance with other embodiments illustrated in figures 6a, 6b, 7-9, the dispensing body 10 is preferably provided with an applicator 19, preferably spongy, by means of which it is possible to apply the mixed substance 26 thus obtained directly on the patient.

The closed end 52 adjoins the mixing chamber 9 so that the dispensing body 10 or the closing element 11 is in direct contact with the mixing substance 26 made and contained in the mixing chamber 9.

The perforating element 7 is arranged inside the main body 5, in a substantially intermediate position between the housing area 8 and the mixing chamber 9.

In detail, the perforating element 7 extends straddling the two areas, in an intermediate portion which acts partly as both housing and for mixing.

The perforating element 7 comprises a punch 71 having a tip 70 facing the open end 51 of the main body 5, facing and thus turned towards the septum 4 of the vial 2, and a base 72, from which the punch 71 is projected, internally connected to the main body 5, in particular to the inner walls of the main body 5.

When the system is not in use, the at least one capsule 6 is retained in the housing area in a distanced position with respect to the perforating element 7, while it is brought against the perforating element 7, which pierces it, when the system is in use. The punch 71 therefore has a height or an extension along the longitudinal axis X of the main body 5 such as to be able to perforate, successively, also several capsules in addition to the septum 4 of the vial: therefore, the height of the due can be, preferably, comprised between 10 mm and 20 mm; the ratio between the diameter of the base and the height of the punch is preferably about 1:3. These should be understood merely as examples, and therefore as indicative and not limiting values. Other suitable dimensional ratios may be selected for the product which is the subject matter of the present invention.

Advantageously, the perforating element 7, in particular the punch 71, comprises at least three spear-shaped fins 711 angularly equally spaced apart from each other with reference to the circular base 72 and sloping towards the tip of the punch 71.

The base 72 is thus preferably provided with at least one opening 721. Preferably, such openings 721 are in a number corresponding to the number of fins 711; such lower openings 721 of the base 72, comprised and delimited between two successive fins 711, are used to make the mixed substance 26 flow out towards the dispensing body 10 or the pierceable membrane 11. Such openings may have larger or smaller surfaces so as to adjust the speed of the outflow of the mixed substance 26.

The special and elongated shape of the punch 71 was developed so as to allow, once the system has been activated, the simultaneous opening of the capsules 6 containing the secondary components to be mixed and, subsequently, the opening of the vial 2 containing the main solution.

Most of the mixing chamber 9 extends in the part below the punch 71, which extends up to an end part 12 of the device 100, viz., up to the closed end 52 of the main body 5.

The openings 721 allow the at least partially mixed substance to flow into the end part 12 of the device 100, in the mixing chamber 9, to complete the mixing.

As mentioned above, the end part 12 of the device 100, viz., the part located at the closed end 52 of the main body 5, serves not only as a mixing chamber 9 since, comprising a dispensing body 10, it can also be used for dosing the mixture directly into body cavities, onto application surfaces or to feed other devices. Finally, such an end part can constitute the support base for further materials such as sponges, absorbent materials, bandages/garments, porous materials so as to facilitate the application of the mixture obtained on the skin and/or surfaces to be treated, as depicted in figures 6a, 6b, 7, 8 and 9.

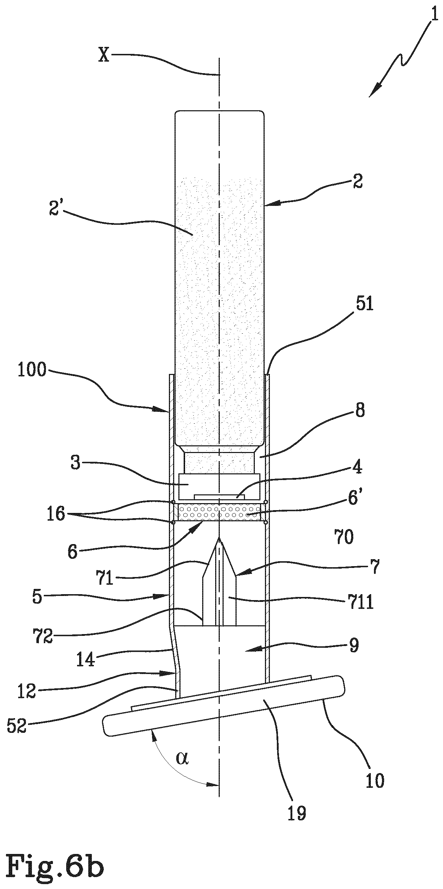

The dispensing body 10 may therefore have a tilting support 14 (figure 6a, 6b), provided with a spongy element or spongy applicator 19 to ease the application of the mixture. Such an applicator 19 is inclined with respect to a longitudinal axis X of the device 100 by an angle α comprised between 30° and 50°. The tilting support 14 allows the applicator 19 to further incline by an angle comprised between 10° and 50° with respect to the axis X.

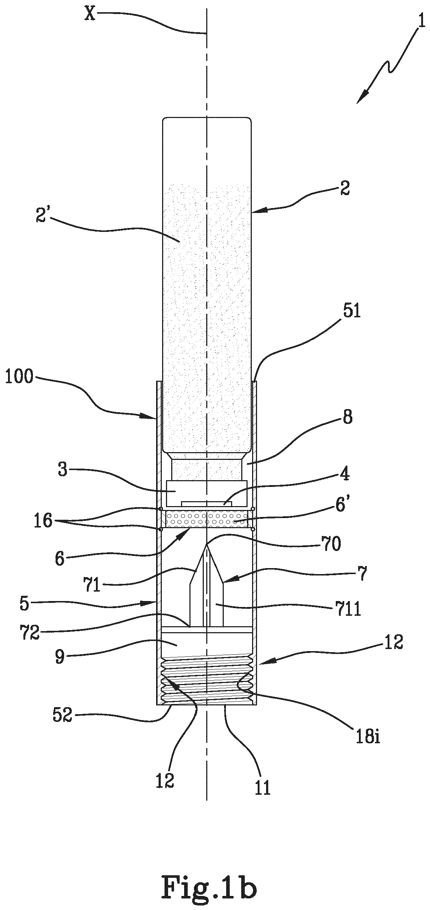

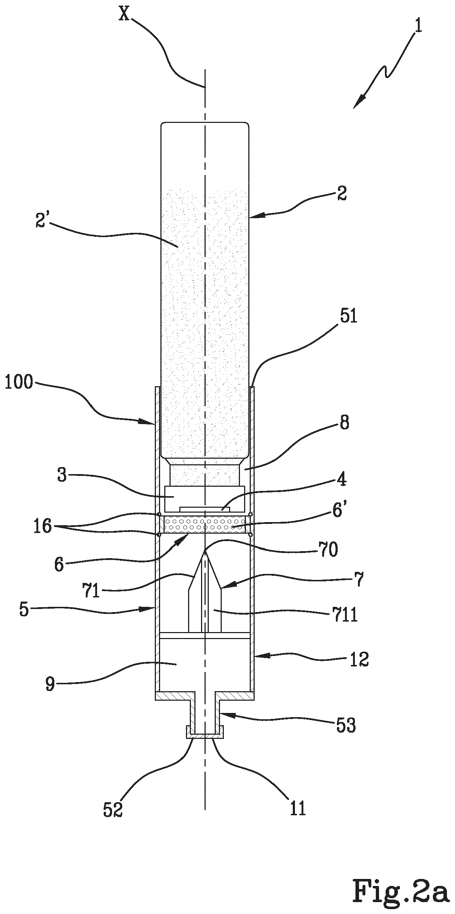

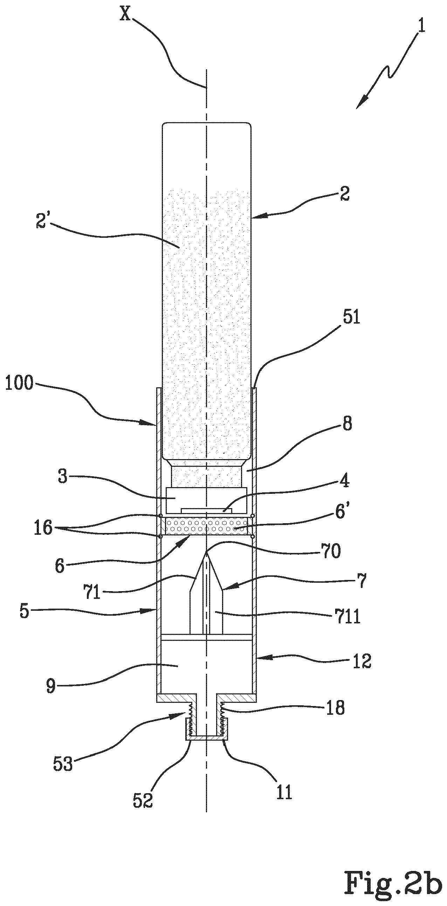

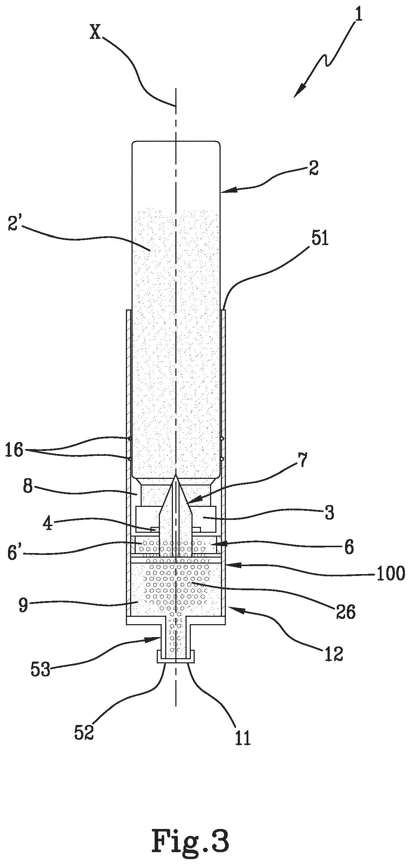

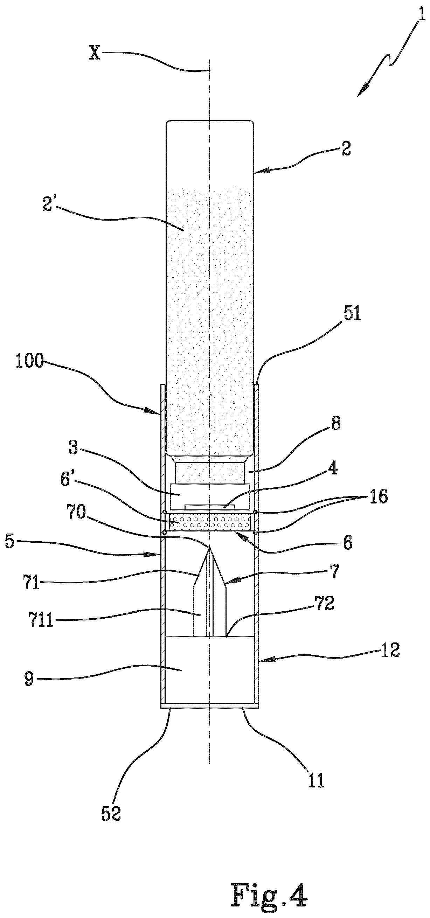

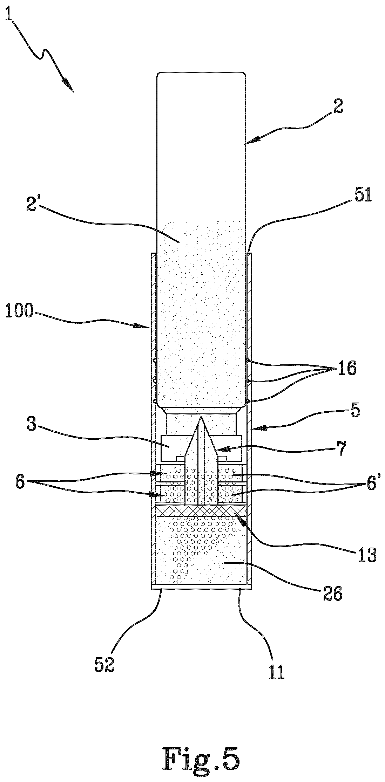

Alternatively, the end part 12 may have, instead of the dispensing body, a closing element 11 such as a flap, a lid, a small cap (screwable as in figure 2b or press-on as in figures 2a and 3), a removable film (as in figures 1a, 1b), a pierceable septum or membrane (as in figures 4 and 5), so that the mixed substance 26 can be withdrawn directly from the mixing chamber 9 closest to the end part 2 of the device 100. In this case, the end part 12 may have a parallelepiped shape, with the closed end 52 orthogonal to the longitudinal axis X, or it may be inclined and have the closed end 52 inclined with respect to the longitudinal axis X.

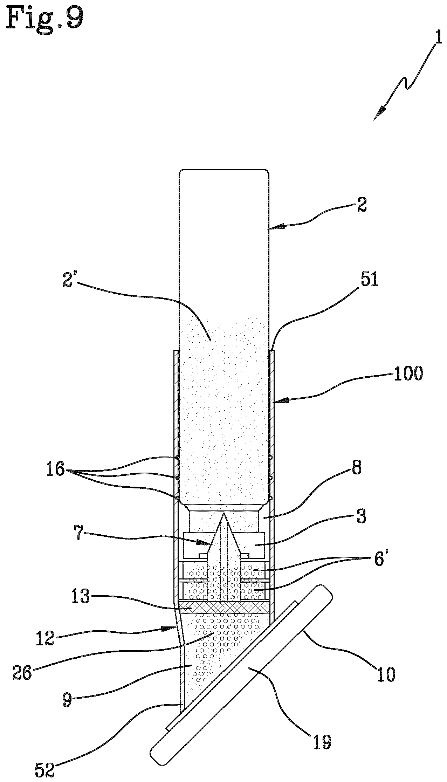

The device 100 may further comprise a porous filter 13 (illustrated, for example, in figures 5 and 9), which can be positioned below the base 72 of the perforating element 7, inside the mixing chamber 9, to decrease or adjust the flow of the mixed substance 26 exiting.

Figures 1a and 1b illustrate two first embodiments which provide a threaded connection 18 (18i and 18e) at the end part 12 of device 100; figure 1a shows a configuration in which the threaded connection 18e is outside the device, so that the outer surface of the end part 12 is threaded. Figure 1b instead shows an alternative configuration where the threaded connection 18i is inside the end part 12, therefore made on the inner surface of the end part 12.

These two alternatives allow the device in question to be used with other devices having threaded connectors.

Figures 2a, 2b and 3 show configurations which provide a closed end 52 having a neck 53. Such a neck is closed by a closing element 11 which may be in the form of a lid, cap or removable film. A variant is illustrated in figure 2b where a thread 18 around the neck 53 is shown; in the illustrated figure the threaded connection 18e is external.

Figures 5 and 6 instead illustrate a variant where the closing element 11 is in the form of a removable film or pierceable septum.

The system 1 may comprise one or more capsules 6 which can be inserted inside the device 100, each containing a respective secondary substance 6' to be mixed with the main substance contained in the vial 2. The secondary substance(s) 6' may be in powdered, granular or even liquid form.

The secondary substance 6' may comprise activating substances, substances which are not compatible when pre-mixed with the main substance, colouring substances or pigments (also not compatible - in the long term - with the main substance) or other substances necessary to obtain the final solution. Preferably, when there are two or more capsules 6, at least one of them may contain a colouring substance to highlight the release of mixture during use.

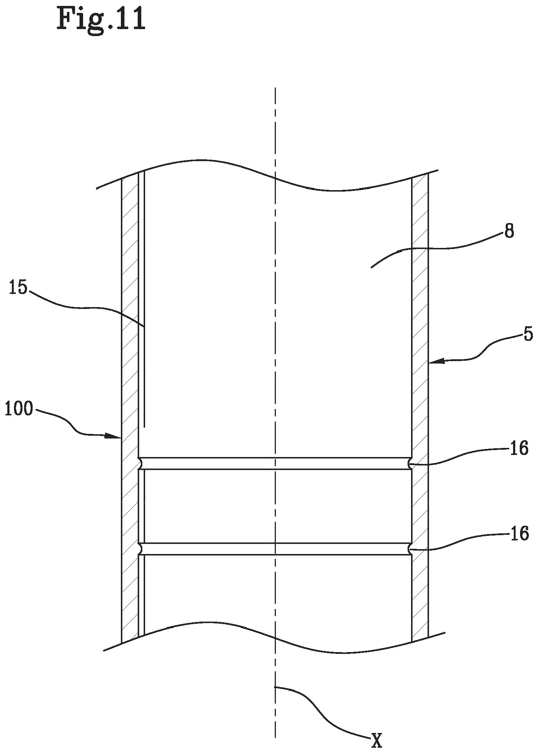

Each capsule 6 preferably has a cylindrical shape delimited by two discs closed laterally by a ring welded at the top and bottom. The ring may provide grooves on the outer surface to facilitate its insertion and guidance within the main body 5. In turn, the main body 5 can provide, on its inner surface, ribs 15 (better visible in figure 11 in which an enlargement of a detail of the main body 5 alone has been depicted) extending longitudinally along the axis X, which act as guide rails for the capsules 6, so that the latter are inserted, slid and oriented correctly, with the bases orthogonal to the longitudinal axis X. The ribs 15 extend, advantageously, along the housing area 8.

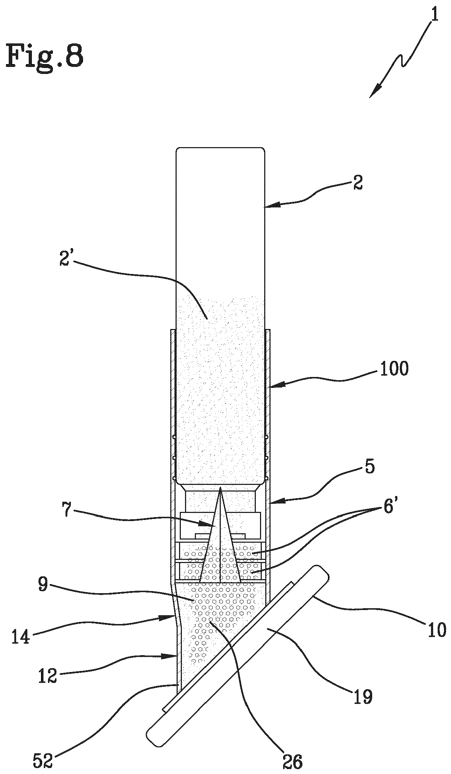

Advantageously, the inner surface of the main body 5, in particular of the housing area 8 is, moreover, provided with thickening elements 16 or small rings (best seen in figure 8) which make horizontal stops, preferably undercut, to hold and stop the capsules 6 and the vial 2 in the correct position in the initial pre-activation and final post-activation positions. The capsules 6 and the vial 2 are thus kept at a distance from the perforating element 7, in particular from the tip 70 of the punch 71 when the system is not in use, therefore until it is activated.

Such rings 16 are sized so as to allow - with a slight pressure of the operator on the base of the vial 2 during the activation of the system - to overcome the undercut to reach the final activation position. The vial, pushed by the operator further inside the device, presses against one or more capsules, which crush the small rings 16 outwards. Once overcome, the capsule(s) face the perforating element which pierces the capsule(s) and the septum of the vial.

In particular, the small stop ring 16 of the vial acts on the shoulder of the vial 2 itself, creating a stop (pre-activation position) inside the main body 5 and determining the correct positioning of the vial within the main body 5 above the perforating element 7.

The bases of the capsules are made of pierceable material and the particular feature of their stackability inside the main body 5 facilitates the multiple perforation of the individual capsules inserted. Once perforated, the stacked capsules 6 also act as a mixing zone for the main substance 2' (contained in the vial 2) with the secondary activation substances 6' (contained in the capsules 6). Such a function allows the operator to create a desired mixture and dosage of the substances to be mixed (very useful especially in the pharmaceutical sector).

Therefore, as long as the system is not activated, the small rings 16 are capable of keeping both the vial and the at least one capsule at a distance from the perforating element, avoiding accidental perforations or mixing of the substances before the actual use.

The system which is the subject matter of the present invention may have various combinations of the number of capsules 6 present (one or more), the presence or absence of the porous filter 13, the shape with dispensing body 10 or with closing element 11. Only a few of the possible combinations of the system which is the subject matter of the present invention have been depicted in the attached figures.

The system and the device which are the subject matter of the present invention achieve the proposed aims and overcome the drawbacks of the prior art.

In fact, the vial may contain solutions, medicines or drugs in a liquid or semi-solid state, and the capsules contain secondary substances which must be mixed with the main substance contained in the vial but which, for reasons of instability or perishability, cannot be mixed with each other except at the time of actual use. Once activated, the system allows the mixing of a main solution (contained in the vial) with any solutions and/or powders contained in the special capsules housed inside the main cylindrical body which externally wraps around the vial like a cap: the use of capsules containing secondary substances to be mixed with the main one ensures a precise dosing and optimal preservation of all the substances over time. Such a device allows to obtain a system which is capable of keeping substances separate which cannot be stored pre-mixed for a long time because they are unstable or not compatible in the long term. With the device, which is the subject matter of the present invention, the mixing only occurs at the time of use, thus ensuring the perfect preservation and compatibility of the individual substances.

The substances contained in the capsules may be used to activate the main formulation or may consist of dyes, medicines or substances which, when mixed with the main solution, provide a synergistic effect with respect to the unmixed individual components. Such substances may take the form of powders, gels, granules or liquids.

The unique features of the device include the possibility of constructing the mixture simply by introducing - in the cylindrical main body - the capsules containing the further substances before activating the device. In fact, starting from a 'base' formulation contained in the main vial, the device allows, for example, the colouring of the main substance using pigments which are not compatible with the substance itself, the addition of a further active ingredient to the main formulation or the introduction of adjuvants and/or synergising agents, etc. The use of several capsules at the same time allows to mix several substances and also in different states (powders, gels, liquids with different viscosities). The preparation of the final mixture is therefore very easy and facilitated for even an inexperienced operator.

The fact that the capsules are separated from the main vial also substantially facilitates the production and assembly process of the device: in fact, it is possible to produce a standard formulation which - thanks to a simple assembly - can subsequently be customised simply by inserting capsules.

Furthermore, the device which is the subject matter of the present invention allows a standard pharmaceutical container to be used without the need to crush the container during the activation of the system, which can cause hazards for the operator and end user.

The capsules can therefore activate, modify, colour, and supplement the basic formulation according to the final intended recipe, therefore the device allows several functions to be achieved simultaneously (e.g., activation of the formulation, colouring, mixing of two or more different powders or medical substances) through a single act of activating the device.

The capacity of the individual capsules can vary according to uses and allows for precise dosages of the contained substances.

The special shape of the punch not only allows an easy opening of the main container but also the correct mixing of the substances contained in the capsule(s) and their complete use. In fact, the special open shape allows the substances to be collected, mixed and channelled towards the lower end part, exploiting 100% of the quantity contained.

The end zone with a tilting support eases the application of the medicine on the surface to be treated. In particular, this shape allows to dose the medicine effectively on non-planar surfaces, which in the case of the human body can be the limbs, neck, armpits, interdigital spaces, groin and all parts/surfaces in general which have a complex shape, or eases the correct application even in a tilted position (180°) or at 90° in all those cases where it is not possible to rotate patients or parts of the body (e.g., in the complete preparation of the lower limbs).

Finally, the particular shape of the punch and the perforation mechanism of the device allows, once the system has been activated, to also be used in a tilted position as the mixed substance is collected in the lower part of the container, preventing the substance from returning to the primary container.

## Claims

1. Device for mixing a main medical substance with a secondary substance comprising:
- an internally hollow main body (5) having an open end (51), through which a hermetically sealed vial (2) containing a main medical substance can be inserted, and a closed end (52), opposite the open end, comprising a dispensing body (10) or a closing element (11) which can be opened; said main body (5) comprising at least one housing area (8) within which said vial (2) can be inserted and at least one capsule (6) containing a secondary substance to be mixed with the main substance; said vial (2) having a closure (3) provided with a pierceable septum (4);
- a perforating element (7), arranged inside said main body (5) to perforate said at least one capsule (6) of a secondary substance and the septum (4) of said vial (2);
- said main body (5) further comprising, therein, a mixing chamber (9), located between the perforating element (7) and the closed end (52), in which the secondary substance is completely mixed with said main substance to make a mixed substance (26); said main body (5) further comprising, in said housing area (8), thickening elements (16) adapted to make temporary stops to keep said at least one capsule (6) in a position distant from said perforating element (7).

2. Device according to the preceding claim, **characterised in that** said closed end (52) adjoins said mixing chamber (9) so that the dispensing body (10) or the closing element (11) are in direct contact with the mixed substance (26) contained in the mixing chamber (9).

3. Device according to claim 1 or 2, **characterised in that** said perforating element (7) comprises a base (72) having at least one opening (721) to make the mixed substance (26) comprising the main medical substance (2') and the secondary substance (6') flow out towards the dispensing body (10) or the closing element (11).

4. Device according to any one of the preceding claims, **characterised in that** said housing area (8) has longitudinal ribs (15) made inside the main body (5), along an inner surface, to enable the correct positioning of the at least said capsule and said vial therein.

5. Device according to any one of the preceding claims, **characterised in that** it comprises a porous filter (13) to adjust the outlet flow of the mixed substance (26); said porous filter (13) being preferably positioned at the base of the perforating element within the mixing chamber (9).

6. Device according to claim 1, **characterised in that** said dispensing body (10) comprises a spongy applicator (19).

7. Device according to the preceding claim, **characterised in that** said dispensing body (10) has a tilting support (14) to ease the application of the mixture.

8. Device according to the preceding claim, **characterised in that** said spongy applicator (19) is inclined with respect to a longitudinal axis (X) of said device by an angle (α) of between 30° and 50°; said tilting support (14) being able to further incline the dispensing body (10), with respect to the starting position, by an angle of between 10° and 50°.

9. Device according to claim 1, **characterised in that** said closing element (11) may be selected from the group comprising a pierceable membrane, a flap, a lid, a small screwable or press-on cap, a removable film, a pierceable septum or membrane.

10. Device according to claim 1, **characterised in that** said main body (5), near said closed end (52) may have a threaded connection (18).

11. System for mixing a main medical substance with a secondary substance comprising:
- a vial (2) containing a main medical substance, hermetically sealed and having a closure (3) provided with a pierceable septum (4);
- at least one capsule (6) containing a secondary substance to be mixed with the main substance;
- a device (100) for mixing a main medical substance with a secondary substance according to one or more of the preceding claims;
wherein said vial (2) and said at least one capsule (6) are inserted into said housing area (8) through said open end (51);
said at least one capsule (6) being retained by the thickening elements (16) present in said housing area (8); said thickening elements (16) are adapted to make temporary stops to keep said at least one capsule (6) at a distance from the perforating element (7) when the system is not in use, while also keeping the vial (2) at a distance from the perforating element (7), and such as to allow disengagement and sliding of said at least one capsule (6) and said vial (2) towards the perforating element (7) to be perforated, when the system is in use.

12. System according to the preceding claim, **characterised in that** it comprises at least two capsules (6), at least one of the two containing a colouring substance to highlight the release of mixture during use.

13. System according to claim 11 or 12, **characterised in that** said main substance (2') is liquid and said secondary substance (6') is powder.
